# EUROPÄISCHE PATENTSCHRIFT

(11) **EP 1 487 475 B1**
(45) Veröffentlichungstag und Bekanntmachung des Hinweises auf die Patenterteilung: **29.09.2010**
(21) Anmeldenummer: 03709761.5
(22) Anmeldetag: 07.03.2003
(51) Int. Cl.: A61K 38/05, A61K 38/55, A61K 31/195, A61P 17/00

(54) **PHARMAZEUTISCHEN ZUBEREITUNGEN VON INHIBITOREN DER AMINOPEPTIDASE N GEGEBENENFALLS IN KOMBINATION MIT INHIBITOREN DER DIPEPTIDYLPEPTIDASE IV ZUR THERAPIE UND PRÄVENTION DERMATOLOGISCHER ERKRANKUNGEN MIT SEBOZYTÄRER HYPERPROLIFERATION UND VERÄNDERTEN DIFFERENZIERUNGSZUSTÄNDEN**
PHARMACEUTICAL PREPARATIONS OF INHIBITORS OF AMINOPEPTIDASE N ALONE OR IN COMBINATION WITH INHIBITORS OF DIPEPTIDYLPEPTIDASE IV FOR THE THERAPY AND PREVENTION OF DERMATOLOGICAL DISEASES WITH SEBORRHOEIC HYPERPROLIFERATION AND ALTERED DIFFERENTIATION STATES
PREPARATIONS PHARMACEUTIQUES DES INHIBITEURS DE L'AMINOPEPTIDASE N SEUL OU EN COMBINAISON AVEC DES INHIBITEURS DE DIPEPTIDYLPEPTIDASE IV POUR TRAITER ET PREVENIR DES MALADIES DERMATOLOGIQUES AVEC HYPERPROLIFERATION DE SEBOCYTES ET ETATS DE DIFFERENCIATION MODIFIES

(30) Priorität: 15.03.2002 DE 10211555
(43) Veröffentlichungstag der Anmeldung: 22.12.2004
(73) Patentinhaber: IMTM GmbH, 39120 Magdeburg (DE); Ansorge, Siegfried, 39291 Hohenwarte (DE); Gollnick, Harald, 39120 Magdeburg (DE); Neubert, Klaus, 06120 Halle (DE); Zouboulis, Christos C., 14195 Berlin (DE)
(72) Erfinder: ANSORGE, Siegfried, 39291 Hohenwarte (DE); GOLLNICK, Harald, 39120 Magdeburg (DE); NEUBERT, Klaus, Martin-Luther-Universität, 06120 Halle (DE); ZOUBOULIS, Christos C., 14195 Berlin (DE); FAUST, Jürgen, Martin-Luther-Universität, 06120 Halle (DE); LENDECKEL, Uwe, Universität Magdeburg Inst., 39120 Magdeburg (DE); REINHOLD, Dirk, Uni. Magdeburg Inst. Immunologie, 39120 Magdeburg (DE); VETTER, Robert, 39120 Magdeburg (DE)
(74) Vertreter: Koepe, Gerd L.
(86) Internationale Anmeldenummer: PCT/EP2003/002356
(87) Internationale Veröffentlichungsnummer: WO 2003/077935

(56) Entgegenhaltungen:
- WO-A-02/053170
- DE-A- 10 025 464
- DE-A- 19 826 972
- AUGUSTYNS K ET AL: "The unique properties of dipeptidyl-peptidase IV (DPP IV/CD26) and the therapeutic potential of DPP IV inhibitors." CURRENT MEDICINAL CHEMISTRY, Bd. 6, Nr. 4, April 1999 (1999-04), Seiten 311-327, XP000870290 ISSN: 0929-8673
- SHIMAZAWA R ET AL: "NOVEL SMALL MOLECULE NONPEPTIDE AMINOPEPTIDASE N INHIBITORS WITH A CYCLIC IMIDE SKELETON" JOURNAL OF ENZYME INHIBITION, NEW YORK, NY, US, Bd. 14, Nr. 4, 1999, Seiten 259-275, XP001018772

## Beschreibung

Die Erfindung beschreibt die Hemmung der für die Proliferation notwendigen DNA-Synthese von Sebozyten durch die Wirkung von Inhibitoren der Aminopeptidase N (APN, EC3.4.11.2, CD13) oder/und der Dipeptidylpeptidase IV (DP IV, EC 3.4.14.5, CD26) im Ergebnis der einzelnen, simultanen oder zeitlich unmittelbar aufeinanderfolgenden Applikation von jeweils spezifischen Inhibitoren dieser Enzyme oder Enzymen gleicher Substratspezifität (APN- oder/und DP IV-analoge Enzymaktivität) auf der Basis von Aminosäurederivaten, Peptiden oder Peptidderivaten, durch welche die Proliferation (DNA-Synthese) von Sebozyten supprimiert wird.

Eine Reihe dermatologischer Erkrankungen gehen mit sebozytärer Hyperproliferation und veränderten Differenzierungszuständen einher. Zu ihnen gehören sowohl benigne follikuläre Hyperproliferationszustände (Akne, akneiforme follikuläre Reaktionen, Steatocystoma multiplex, Talgdrüsen-Naevi, senile Talgdrüsen-hypertrophie, Seborrhoe der Haut und Haare) als auch maligne follikuläre Hyperproliferationszustände (Mischtumoren, Sebaceome, Talgdrüsen-Tumoren, Talgdrüsen-CA).

Peptidasen wie die Dipeptidylpetidase IV und die Aminopeptidase N oder ähnlich wirkende Enzyme sind für die Regulation bzw. Modulation von Wechselwirkungen zwischen Zellen besonders interessant, da sie u. a. als Ektoenzyme in der Plasmamembran der Zellen lokalisiert sind, Interaktionen mit anderen extrazellulären Strukturen eingehen, peptiderge Botenstoffe durch enzymkatalysierte Hydrolyse aktivieren bzw. inaktivieren und dadurch wichtig für die Zell-Zell-Kommunikation sind [Yaron A, et al.: Proline-dependent structural and biological properties of peptides and proteins. Crit Rev Biochem Mol Biol 1993;28:31-81; Vanhoof G, et al.: Proline motifs in peptides and their biological processing. FASEB J 1995;9:736-744].

Es ist gezeigt worden, dass im Prozess der Aktivierung und klonalen Expansion von Immunzellen, insbesondere von T-Lymphozyten, membranständige Peptidasen wie DP IV oder APN eine Schlüsselrolle spielen [Fleischer B: CD26 a surface protease involved in T-cell activation. Immunology Today 1994; 15:180-184; Lendeckel U et al.: Role of alanyl aminopeptidase in growth and function of human T cells. International Journal of Molecular Medicine 1999; 4:17-27; Riemann D et al.: CD13 - not just a marker in leukemia typing. Immunology Today 1999; 20:83-88]. Verschiedene Funktionen Mitogen-stimulierter mononukleärer Zellen (MNZ) oder angereicherter T-Lymphozyten wie DNA-Synthese, Produktion und Sekretion von immunstimulierenden Zytokinen (IL-2, IL-6, IL-12, IFN-γ) und Helferfunktionen für B-Zellen (IgG- und IgM-Synthese) können in Gegenwart von spezifischen Inhibitoren der DP IV oder der APN gehemmt werden [Schön E et al.: The dipeptidyl peptidase IV, a membrane enzyme involved in the proliferation of T lymphocytes. Biomed. Biochim. Acta 1985; 2: K9-K15; Schön E et al.: The role of dipeptidyl peptidase IV in human T lymphocyte activation. Inhibitors and antibodies against dipeptidyl peptidase IV suppress lymphocyte proliferation and immunoglobulin synthesis in vitro. Eur. J. Immunol. 1987; 17: 1821-1826; Reinhold D et al.: Inhibitors of dipeptidyl peptidase IV induce secretion of transforming growth factor β1 in PWM-stimulated PBMNC and T cells. Immunology 1997; 91: 354-360; Lendeckel U et al.: Induction of the membrane alanyl aminopeptidase gene and surface expression in human T-cells by mitogenic activation. Biochem. J. 1996; 319: 817-823; Kähne T et al.: Dipeptidyl peptidase IV: A cell surface peptidase involved in regulating T cell growth (Review). Int. J. Mol. Med. 1999; 4: 3-15; Lendeckel U et al.: Role of alanyl aminopeptidase in growth and function of human T cells (Review). Int. J. Mol. Med. 1999; 4: 17-27]. Es ist bereits bekannt, daß die Behandlung von Autoimmunerkrankungen und Transplantatabstoßung durch Hemmung der auf Immunzellen lokalisierten Dipeptidylpetidase IV mit Hilfe von synthetischen Inhibitoren möglich ist (z. B. EP764151 A1, WO 9529691, EP731789 A1, EP528858).

Der Erfindung liegt der überraschende Befund zugrunde, dass die einzelne oder gleichzeitige Wirkung von Inhibitoren der auf bzw. in Sebozyten exprimierten Dipeptidylpeptidase IV/CD26 und Aminopeptidase N/CD13 oder Enzyme gleicher Substratspezifität (APN- oder/und DP IV-analoge Enzymaktivität), die Proliferation (DNA-Synthese) dieser Zellen hemmt.

Die vorliegende Erfindung betriftt die Verwendung von Inhibitoren der Alanyl-Aminopeptidase (Aminopeptidase N, APN) oder von Inhibitoren von Enzymen gleicher Substratspezifität (APN-analoger Enzymaktivität) zur Herstellung pharmazeutischer Zubereitungen zur Vorbeugung und Therapie von benignen follikulären Hyperproliferationszuständen oder malignen follikulären Hyperproliferationszuständen, die mit einer sebozytären Hyperproliferation und veränderten Differenzierungszuständen einhergehen, worin die Inhibitoren der APN ausgewählt sind aus der Gruppe, die besteht aus Actinonin, Leuhistin, Phebestin, RB3014, Amastatin, Bestatin (Ubenimex), Probestin, ß-Aminothiole, α-Aminophosphin-Säuren und deren Salze.

Aüßerdem betrifft die vorliegende Erfindung die Verwendung der o.g. APN-Inhibitoren in Kombination mit Inhibitoren der Dipeptidylpeptidase IV (DP IV) oder von Inhibitoren von Enzymen mit gleicher Substratspezifität (DP IV-analoger Enzymaktivität), worin die Inhibitoren der DP IV ausgewählt sind aus der Gruppe, die besteht aus
- Xaa-Pro-Dipeptide oder deren Salze,
   worin Xaa eine α-Aminosäure bzw. ein seitenkettengeschütztes Derivat ist,
- Xaa-Xaa-(Trp)-Pro-(Xaa)n-Peptide oder deren Salze,
   worin Xaa eine α-Aminosäure ist und n = 0 bis 10 ist,
- Aminosäure(Xaa)-amide oder deren Salze,
   wobei Xaa eine α-Aminosäure bzw. ein seitenkettengeschütztes Derivat ist und cyclische Amine als Amidstruktur fungieren,
- Tryptophan-1,2,3,4-tetrahydroisochinolin-3-carbonsäurederivate (TSL), und
- [(2S,2S',2S")-2-[2'-[2"-Amino-3"-(indo)-3'"-yl)-1"-oxopropyl]-1',2',3',4'-tetrahydro-6;8'-dihydroxy-7-methoxyisochinol-3-yl-carbonyl-amino]-4-hydroxymethyl-5-hydroxypentansäure] (TMC-2A).

Unsere Erfindung zeigt, dass zur Therapie und zur Prävention von dermatologischen Erkrankungen mit sebozytärer Hyperproliferation und veränderten Differenzierungszuständen (benigne follikuläre Hyperproliferationszustände wie Akne, akneiforme follikuläre Reaktionen, Steatocystoma multiplex, Talgdrüsen-Naevi, senile Talgdrüsenhypertrophie, Seborrhoe der Haut und Haare und SAHA-Syndrom Seborrhoe, Akne, Hirsütismüs, Alopecie als auch maligne follikuläre Hyperproliferationszustände wie Mischtumoren, Sebaceome, Naevus sebaceus mit maligner Entwicklung Talgdrüsen-Tumoren, Talgdrüsen-CA), für deren Entstehung die Proliferation von Sebozyten eine zentrale Bedeutung hat, die einzelne oder gleichzeitige Applikation von Hemmstoffen der DP IV und der APN oder Enzymen gleicher Substratspezifität (APN- oder/und DP IV-analoge Enzymaktivität) bzw. entsprechender Zubereitungen und Darreichungsformen daraus geeignet sind.

Im einzelnen liegen der Erfindung die Befunde zugrunde, dass die DNA-Synthese von Sebozyten durch die Gabe von Inhibitoren der Dipeptidylpeptidase IV oder/und der Aminopeptidase N signifikant inhibiert wird.

Die oben genannten Erkrankungen werden bisher topisch und/oder systemisch mit Antibiotika und/oder antiproliferativen und differenzierenden Substanzen (Antiandrogenen, 13-cis-Retinsäure u. a) behandelt. Insbesondere bei der systemischen Anwendung treten häufig unerwünschte Nebenwirkungen auf. Dies sind u. a. Teratogenität, Lipidstoffwechselstörungen, psychoreaktive Erscheinungen, gastrointestinale Beschwerden sowie muco-cutane irritative Reaktionen.

Der Einsatz von DP IV- oder/und APN-Inhibitoren würde bei den genannten Erkrankungen eine gänzlich neuartige, vorraussichtlich sehr effektive, möglicherweise kostengünstige Therapieform und einen wertvollen alternativen Bestandteil der bestehenden Therapiekonzepte darstellen.

Die erfindungsgemäß applizierten Inhibitoren der Dipeptidylpeptidase IV oder/und der Aminopeptidase N oder Enzymen gleicher Substratspezifität (APN- oder/und DP IV-analoge Enzymaktivität) können in pharmazeutisch anwendbaren Formulierungs-komplexen als Inhibitoren, Substrate, Pseudosubstrate, inhibitorisch wirkende Peptide und Peptidderivate dieser Enzyme zur Anwendung kommen.

Bevorzugte Effektoren sind beispielsweise für die DP IV Xaa-Pro-Dipeptide, entsprechende Derivate, vorzugsweise Dipeptidphosphonsäurediarylester, und deren Salze, Dipeptidboronsäuren (z.B. Pro-boro-Pro) und deren Salze, Xaa-Xaa-(Trp)-Pro-(Xaa)n-Peptide (n=0-10), entsprechende Derivate und deren Salze bzw. Aminosäure (Xaa)-amide, entsprechende Derivate und deren Salze, wobei Xaa eine α-Aminosäure/Iminosäure bzw. ein α-Aminosäurederivat/Iminosäurederivat, vorzugsweise N^{ε}-4-Nitrobenzyl-oxycarbon-yl-L-Lysin, L-Prolin, L-Tryptophan,L-Isoleucin, L-Valin ist und als Amidstruktur cyclische Amine, z.B. Pyrrolidin, Piperidin, Thiazolidin und deren Derivate, Tryptophan-1,2,3,4-tetrahydroisochinolin-3-carbonsäurederivate (TSL) und/oder (2S,2S',2S")-2-[2'-[2"-Amino-3"-(indol-3'''-yl)-1 "-oxoprolyl]-1',2',3', 4'-tetrahydro-6'8'-dihydroxy-7-methoxyisochinol-3-yl-carbonyl-amino]-4-hydromethyl-5-hydropentansäure (TMC-2A). Derartige Verbindungen und deren Herstellung wurden in einem früheren Patent beschrieben (K. Neubert et al. DD296075A5). Bevorzugte Inhibitoren für die Alanyl-Aminopeptidase sind Bestatin (Ubenimex), Actinonin, Probestin, Phebestin, RB3014, Leuhistin, Amastatin, ß-Aminothiole, α-Aminophosphinsäuren, α-Aminophosphinsäurederivate bevorzugt D-Phe-_{ψ}[PO(OH)-CH₂]-Phe-Phe und deren Salze.

Die Inhibitoren oder pharmazeutische Zubereitungen, die diese enthalten, werden simultan mit bekannten Trägerstoffen verabreicht. Die Verabreichung erfolgt einerseits als topische Applikation in Form von z.B. Cremes, Salben, Pasten, Gelen, Lösungen, Sprays, Lipo- und Nanosomen,

Schüttelmixturen, Hydrokolloidverbänden, Pflaster und ähnliche neue Trägersubstrate, Jet-Injektion bzw. anderen dermatologischen Grundlagen/Vehikeln einschließlich instillativer Applikation und andererseits als systemische Applikation zur oralen, transdermalen, intravenösen, subcutanen, intracutanen, intramuskulären Anwendung in geeigneten Rezepturen bzw. in geeigneter Galenik.

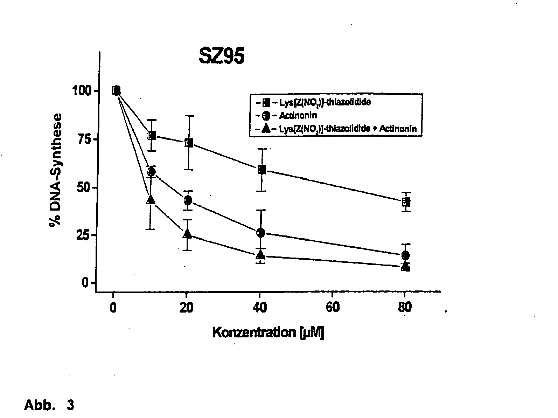

In dem Beispiel wird auf Abbildungen 1 bis 3 Bezug genommen. Diese zeigen: Abbildung 1 den durchflußzytemetrischen Nachweis der Expression von DP-IV (CD26) und APN (CD13) auf SZ95-Zellen; Abbildung 2 den Nachweis der Expression von DP-IV (CD26) und APN (CD13) auf SZ95-Zellen mittels RT-PCR; und Abbildung 3 den dosisabhängigen Effekt von Inhibitoren der DP IV (Lys[Z(NO₂)]-thiazolidid) und der Aminopeptidase N (Actinonin) auf die DNA-Synthese humaner SZ95 Sebozyten.

### Ausführungsbeispiel 1

### Inhibierung der DNA-Synthese der immortalisierten humanen Sebozytenzelllinie SZ95 durch Inkubation mit synthetischen Inhibitoren der DP IV oder/und der APN

Unsere Untersuchungen zeigen, dass die DNA-Synthese der immortalisierten humanen Sebozytenzelllinie SZ95 (Zouboulis C.C. et al: Establishment and characterization of an immortalized human sebaceous gland cell line (SZ95). J. Invest. Dermatol. 1999, 113: 1011-1020) durch die Administration von Inhibitoren der DP IV (Lys[Z(NO₂)]-thiazolidid) oder/und der APN (Actinonin) dosisabhängig gehemmt wird.

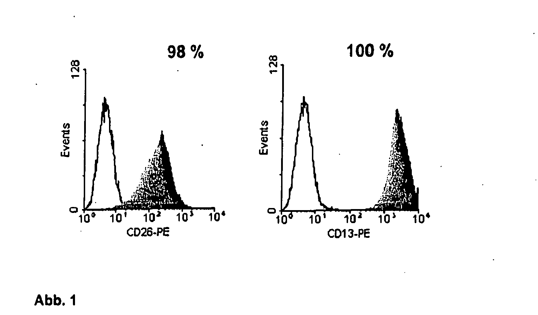

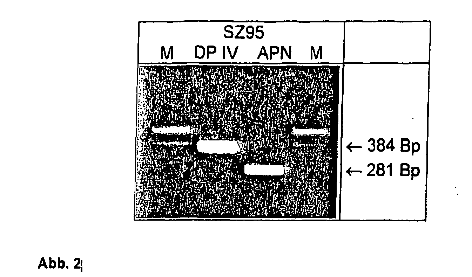

Die humane Sebozytenzelllinie SZ95, welche als Zellmodell für die Akne akzeptiert ist, exprimiert stark DP IV und APN (Abb. 1). Die Enzymaktivität der DP IV von vitalen Zellen beträgt 38 ± 18 pkat/10⁶ Zellen, die der APN beträgt 262 ± 58 pkat/10⁶ Zellen (n = 3). Entsprechend ist die mRNA von APN und DP IV auf diesen Zellen nachweisbar (Abb. 2).

SZ95-Zellen wurden 48 h mit den oben genannten Inhibitoren inkubiert und anschließend über die Messung der ³[H]-Thymidin-Inkorporation die DNA-Synthese bestimmt, wie bei Reinhold et al. beschrieben (Reinhold D et al.: Inhibitors of dipeptidyl peptidase IV induce secretion of transforming growth factor β1 in PWM-stimulated PBMNC and T cells. Immunology 1997; 91: 354-360). Abbildung 2 zeigt die dosisab-hängige Hemmung der DNA-Synthese.

Die Zellen wurden über 48 Stunden mit den angegebenen Konzentrationen der Inhibitoren inkubiert. Anschließend wurde dem Kulturmedium ³[H]-Methyl-Thymidin zugesetzt und nach weiteren 6 Stunden die in die DNA eingebaute Menge an ³[H]-Thymidin gemessen.

## Patentansprüche

1. Verwendung von Inhibitoren der Alanyl-Aminopeptidase (Aminopeptidase N, APN) oder von Inhibitoren von Enzymen gleicher Substratspezifität (APN-analoger Enzymaktivität) zur Herstellung pharmazeutischer Zubereitungen zur Vorbeugung und Therapie von benignen follikulären Hyperproliferationszuständen oder malignen follikulären Hyperproliferationszuständen, die mit einer sebozytären Hyperproliferation und veränderten Differenzierungszuständen einhergehen, worin die Inhibitoren der APN ausgewählt sind aus der Gruppe, die besteht aus Actinonin, Leuhistin, Phebestin, RB3014, Amastatin, Bestatin (Ubenimex), Probestin, ß-Aminothiole, α-Aminophosphin-Säuren und deren Salze.

2. Verwendung nach Anspruch 1, worin die α-Aminophosphinsäure D-Phe-ψPO (OH)-CH₂]-Phe-Phe ist.

3. Verwendung nach Anspruch 1 oder Anspruch 2 in Kombination mit Inhibitoren der Dipeptidylpeptidase IV (DP IV) oder von Inhibitoren von Enzymen mit gleicher Substratspezifität (DP IV-analoger Enzymaktivität), worin die Inhibitoren der DP IV ausgewählt sind aus der Gruppe, die besteht aus
- Xaa-Pro-Dipeptide oder deren Salze,
worin Xaa eine α-Aminosäure bzw. ein seitenkettengeschütztes Derivat ist,
- Xaa-Xaa-(Trp)-Pro-(Xaa)n-Peptide oder deren Salze,
worin Xaa eine α-Aminosäure ist und n = 0 bis10 ist,
- Aminosäure(Xaa)-amide oder deren Salze,
wobei Xaa eine α-Aminosäure bzw. ein seitenkettengeschütztes Derivat ist und cyclische Amine als Amidstruktur fungieren,
- Tryptophan-1,2,3,4-tetrahydroisochinolin-3-carbonsäurederivate (TSL), und
- [(2S,2S',2S")-2-[2'-[2"-Amino-3"-(indol-3''-yl)-1"-oxopropyl]-1',2',3',4'-tetrahydro-6; 8'-dihydroxy-7-methoxyisochinol-3-yl-carbonyl-amino]-4-hydroxymethyl-5-hydroxypentansäure](TMC-2A).

4. Verwendung nach Anspruch 3, worin das Xaa-Pro-dipeptid ein Dipeptidphosphonsäurediarylester, eine Dipeptidboronsäure oder deren Salze ist.

5. Verwendung nach Anspruch 4, worin die Dipeptidboronsäure Pro-boro-Pro ist.

6. Verwendung nach Anspruch 3, worin das Xaa des Aminosäure(Xaa)-amids ausgewählt ist aus der Gruppe; die besteht aus N^{ε}-4-Nitrobenzyloxycarbonyl-L-Lysin, L-Isoleucin, L-Valin, L-Tryptophan und L-Prolin.

7. Verwendung nach Anspruch 3, worin das cyclische Amin ausgewählt ist aus der Gruppe, die besteht aus Pyrrolidin, Piperidin und Thiazolidin.

8. Verwendung nach einem der Ansprüche 3, 6 und 7, worin das Aminosäure(Xaa)-amid ausgewählt ist aus der Gruppe, die besteht aus N^{ε}-4-Nitrobenzyl-oxycarbonyl-L-lysin-thiazolidid, N^{ε}-4-Nitrobenzyl-oxycarbonyl-L-lysin-pyrrolidid, N^{ε}-4-Nitrobenzyl-oxycarbonyl-L-lysin-piperidid, N^{ε}-4-Nitrobenzyl-oxycarbonyl-L-lysin-2-cyanothiazolidid, N^{ε}-4-Nitrobenzyl-oxycarbonyl-L-lysin-2-cyanopyrrolidid und N^{ε}-4-Nitrobenzyl-oxycarbonyl-L-lysin-2-cyanopiperidid.

9. Verwendung nach einem der Ansprüche 3 bis 8, worin der DP IV-Inhibitor Lys[Z(NO₂)]-thiazolidid ist, worin Lys für einen L-Lysin-Rest und Z(NO₂) für 4-Nitrobenzyloxycarbonyl steht, und der APN-Inhibitor Actinonin ist.

10. Verwendung nach einem der vorangehenden Ansprüche in Kombination mit an sich bekannten Träger-, Hilfs- und/oder Zusatzstoffen.

11. Verwendung nach einem der vorangehenden Ansprüche, worin die benignen follikulären Hyperproliferationszustände ausgewählt sind aus der Gruppe, die besteht aus Akne, akneiforme follikuläre Reaktionen, Steatocystoma multiplex, Talgdrüsen-Naevi, senile Talgdrüsenhypertrophie, Seborrhoe der Haut und Haare und SAHA-Syndrom [Seborrhoe, Akne, Hirsutismus, Alopecie].

12. Verwendung nach einem der vorangehenden Ansprüche, worin die malignen follikulären Hyperproliferationszustände ausgewählt sind aus der Gruppe, die besteht aus Mischtumoren, Sebaceome, Nävus sebaceus mit maligner Entwicklung, Talgdrüsen-Tumoren, und Talgdrüsen-CA.

13. Verwendung nach einem der Ansprüche 3 bis 12, worin der Inhibitor der Dipeptidylpeptidase IV (DP IV) oder der Inhibitor von Enzymen mit gleicher Substratspezifität (DP IV-analoger Enzymaktivität) und der Inhibitor der Alanyl-Aminopeptidase (Aminopeptidase N, APN) oder der Inhibitor von Enzymen mit gleicher Substratspezifität (APN-analoger Enzymaktivität) in räumlich getrennter Formulierung gleichzeitig oder zeitlich unmittelbar aufeinanderfolgend mit dem Ziel einer gemeinsamen Wirkung zu verabreichen ist.

14. Verwendung nach einem der vorangehenden Ansprüche für die systemische Anwendung zur oralen, transdermalen, intravenösen, subcutanen, intracutanen, intramuskulären, rektalen, vaginalen, oder sublingualen Applikation.

15. Verwendung nach einem der vorangehenden Ansprüche für die topische Anwendung in Form von z. B. Cremes, Salben, Pasten, Gelen, Lösungen, Sprays, Lipo- bzw. Nanosomen, Schüttelmixturen, Hydrokolloidverbänden bzw. anderen dermatologischen Grundlagen/Vehikeln, einschließlich instillativer Applikation.

## Claims

1. A use of inhibitors of alanyl-aminopeptidase (aminopeptidase N, APN) or of inhibitors of enzymes having a similar substrate specificity (APN-analogous enzymatic activity) for the preparation of pharmaceutical preparations for the prevention and therapy of benign follicular hyperproliferation conditions or malign follicular hyperproliferation conditions associated with sebaceous hyperproliferation and modified states of differentiation, wherein the inhibitors of APN are selected from the group consisting of actinonin, leuhistin, phebestin, RB3014, amastatin, bestatin (ubenimex), probestin, ß-aminothiols, α-aminophosphinic acids and salts thereof.

2. The use according to claim 1, wherein the α-aminophosphinic acid derivative is D-Phe-ψ-[PO(OH)-CH₂]-Phe-Phe.

3. The use according to claim 1 or claim 2 in combination with inhibitors of dipeptidyl peptidase IV (DP IV) or inhibitors of enzymes having similar substrate specificity (DP IV analogous enzymatic activity), wherein the inhibitors of DP IV are selected from the group consisting of
- Xaa-Pro-dipeptides or salts thereof,
wherein Xaa is an α-amino acid or a side chain protected derivative thereof;
- Xaa-Xaa-(Trp)-Pro-(Xaa)ₙ peptides or salts thereof,
wherein Xaa is an α-amino acid and n = 0 to 10;
- amino acid(Xaa)-amides or salts thereof,
wherein Xaa is an α-amino acid or a side chain protected derivative thereof and wherein the amide structure is a cyclic amine;
- tryptophane-1,2,3,4-tetrahydroisoquinoline-3-carboxylic acid derivatives (TSL); and
- [(2*S*,2*S'*,2*S"*)-2-[2'-[2"-amino-3"-(indol-3"'-yl)-1"-oxopropyl]-1',2',3',4'-tetrahydro-6',8'-dihydroxy-7'-methoxyisoquinol-3-yl-carbonylamino]-4-hydroxymethyl-5-hydroxypentanoic acid] (TMC-2A).

4. The use according to claim 3, wherein the Xaa-Pro-dipeptide is a dipeptide phosphonic acid diaryl ester or a dipeptide boronic acid or salts thereof.

5. The use according to claim 4, wherein the dipeptide boronic acid is Pro-Boro-Pro.

6. The use according to claim 3, wherein the Xaa of the amino acid(Xaa)-amide is selected from the group consisting of N'-4-nitrobenzyloxycarbonyl-L-lysine, L-isoleucine, L-valine, L- tryptophane and L-proline.

7. The use according to claim 3, wherein the cyclic amine is selected from the group consisting of pyrrolidine, piperidine and thiazolodine.

8. The use according to any of the claims 3, 6 and 7, wherein the amino acid(Xaa)-amide is selected from the group consisting of
N^{ε}-4-nitrobenzyloxycarbonyl-L-lysine thiazolidide,
N^{ε}-4-nitrobenzyloxycarbonyl-L-lysine pyrrolidide,
N^{ε}-4-nitrobenzyloxycarbonyl-L-lysine piperidide,
N^{ε}-4-nitrobenzyloxycarbonyl-L-lysine 2-cyanothiazolidide,
N^{ε}-4-nitrobenzyloxycarbonyl-L-lysine 2-cyanopyrrolidide, and
N^{ε}-4-nitrobenzyloxycarbonyl-L-lysine 2-cyano-piperidide.

9. The use according to any of the claims 3 to 8, wherein the DP IV inhibitor is Lys[Z(NO2)]-thiazolodide, wherein Lys is a L-lysine residue and Z(N02) is 4-nitrobenzyloxycarbonyl, and the APN inhibitor is actinonin.

10. The use according to any of the preceding claims in combination with per se known carrier substances, additives and/or auxiliary substances.

11. The use according to any of the preceding claims, wherein the benign follicular hyperproliferation conditions are selected from the group consisting of acne, acneiform follicular reactions, steatocystoma multiplex, naevi of sebaceous glands, senile sebaceous gland hypertrophy, seborrhoea of the skin and of the hair and SAHA syndrome [seborrhoea, acne, hirsutism, alopecia].

12. The use according to any of the preceding claims, wherein the malign follicular hyperproliferation conditions are selected from the group consisting of mixed tumors, sebaceomes, naevus sebaceous with malign development, sebaceous gland tumors and sebaceous gland CA.

13. The use according to any of the claims 3 to 12, wherein the inhibitor of the dipeptidyl peptidase IV (DP IV) or the inhibitor of enzymes having a similar substrate specificity (DP IV-analogous enzymatic activity) and the inhibitor of alanyl-aminopeptidase (aminopeptidase N, APN) or the inhibitor of enzymes having similar substrate specificity (APN-analogous enzymatic activity) in a compartmentally separate formulation are to be administered simultaneously or consecutively in time with the aim of a combined effect.

14. The use according to any of the preceding claims for a systemic application for a oral, transdermal, intravenous, subcutaneous, intracutaneous, intramuscular, rectal, vaginal or sublingual administration.

15. The use according to any of the preceding claims for a topical application in the form of e.g. creams, ointments, pastes, gels, solutions, sprays, liposomes, nanosomes, agitated mixtures, hydro-colloid dressings and other dermatological bases/vehicles, including instillative applications.

## Revendications

1. Utilisation d'inhibiteurs de l'alanyl-aminopeptidase (aminopeptidase N, APN) ou d'inhibiteurs d'enzymes possédant une spécificité de substrat identique (activité enzymatique analogue à celle de l'APN) pour l'obtention de préparations pharmaceutiques destinées à la prévention et à la thérapie d'états d'hyperprolifération folliculaire bénigne ou d'états d'hyperprolifération folliculaire maligne, qui s'accompagnent d'une hyperprolifération sébocytaire et d'états de différenciation modifiés, les inhibiteurs de l'APN étant choisis parmi le groupe qui est constitué par l'actinonine, la leuhistine, la phébestine, RB3014, l'amastatine, la bestatine (Ubenimex), la probestine, les β-aminothiols, les acides α-aminophosphiniques et leurs sels.

2. Utilisation selon la revendication 1, dans laquelle l'acide α-amino-phosphinique est D-Phe-yr[PO(OH)-CH₂]Phe-Phe.

3. Utilisation selon la revendication 1 ou 2, en combinaison avec des inhibiteurs de la dipeptidylpeptidase IV (DP IV) ou d'inhibiteurs d'enzymes possédant une spécificité de substrat identique (activité enzymatique analogue à celle de la DP IV), les inhibiteurs de la DP IV étant choisis parmi le groupe qui est constitué par :
- des dipeptides Xaa-Pro ou leurs sels,
Xaa représentant un acide α-aminé, respectivement un dérivé dont la chaîne latérale est protégée ;
- des peptides Xaa-Xaa-(Trp)-Pro-(Xaa)ₙ ou leurs sels,
Xaa représentant un acide α-aminé et n = 0 à 10 ;
- des amides d'acides aminés(Xaa) ou leurs sels,
Xaa représentant un acide α-aminé respectivement un dérivé dont la chaîne latérale est protégée et des amines cycliques faisant office de structure amide ;
- des dérivés de l'acide tryptophane-1,2,3,4-tétrahydroisoquinoléine-3-carboxylique (TSL) ; et
- l'acide [(2S,2S',2S")-2-[2'-[2"-amino-3"-(indol-3'''-yl)-1"-oxopropyl]-1',2',3',4'-tétrahydro-6',8'-dihydroxy-7-méthoxyisoquinol-3-yl-carbonyl-amino]-4-hydroxyméthyl-5-hydroxypentanoïque] (TMC-2A).

4. Utilisation selon la revendication 3, dans laquelle le dipeptide Xaa-Pro est un ester diarylique de l'acide dipeptidephosphonique, un acide dipeptideborique ou ses sels.

5. Utilisation selon la revendication 4, dans laquelle l'acide dipeptideborique est Pro-boro-Pro.

6. Utilisation selon la revendication 3, dans laquelle le Xaa de l'amide de l'acide aminé(Xaa) est choisi parmi le groupe qui est constitué par la N^{ε}-4-nitrobenzyloxycarbonyl-L-lysine, la L-isoleucine, la L-valine, le L-tryptophane et la L-proline.

7. Utilisation selon la revendication 3, dans laquelle l'amine cyclique est choisie parmi le groupe qui est constitué par la pyrrolidine, la pipéridine et la thiazolidine.

8. Utilisation selon l'une quelconque des revendications 3, 6 et 7, dans laquelle l'amide de l'acide aminé(Xaa) est choisi parmi le groupe qui est constitué par le N^{ε}-4-nitrobenzyloxycarbonyl-L-lysine-thiazolidide, le N^{ε}-4-nitrobenzyl-oxycarbonyl-L-lysine-pyrrolidide, le N^{ε}-4-nitrobenzyl-oxycarbonyl-L-lysine-pipéridide, le N^{ε}-4-nitrobenzyl-oxycarbonyl-L-lysine-2-cyanothiazolidide, le N^{ε}-4-nitrobenzyl-oxycarbonyl-L-lysine-2-cyanopyrrolidide et le N^{ε}-4-nitrobenzyl-oxycarbonyl-L-lysine-2-cyanopipéridide.

9. Utilisation selon l'une quelconque des revendications 3 à 8, dans laquelle l'inhibiteur de la DP IV est le Lys[Z(NO₂)]-thiazolidide, Lys représentant un résidu de L-Lysine et Z(NO₂) représentant un groupe 4-nitrobenzyloxycarbonyle et l'inhibiteur de l'APN représentant l'actinonine.

10. Utilisation selon l'une quelconque des revendications précédentes, en combinaison avec des supports, des adjuvants et/ou additifs connus en soi.

11. Utilisation selon l'une quelconque des revendications précédentes, dans laquelle les états d'hyperprolifération folliculaire bénigne sont choisis parmi le groupe qui est constitué par l'acné, des réactions folliculaires acnéiformes, Steatocystoma multiplex, des naevi des glandes sébacées, l'hypertrophie sénile des glandes sébacées, la séborrhée de la peau et des cheveux et le syndrome SAHA [séborrhée, acné, hirsutisme, alopécie].

12. Utilisation selon l'une quelconque des revendications précédentes, dans laquelle les états d'hyperprolifération folliculaire maligne sont choisis parmi le groupe qui est constitué par des tumeurs mixtes, des sébacéomes, Naevus sebaceus avec développement malin, des tumeurs des glandes sébacées et le carcinome des glandes sébacées.

13. Utilisation selon l'une quelconque des revendications 3 à 12, dans laquelle l'inhibiteur de la dipeptidylpeptidase IV (DP IV) ou l'inhibiteur d'enzymes possédant une spécificité de substrat identique (activité enzymatique analogue à celle de la DP IV) et l'inhibiteur de l'alanyl-aminopeptidase (aminopeptidase N, APN) ou l'inhibiteur d'enzymes possédant une spécificité de substrat identique (activité enzymatique analogue à celle de l'APN) doit être administré dans une formulation séparée dans l'espace, de manière simultanée ou d'une manière directement successive dans le temps dans le but d'obtenir un effet commun.

14. Utilisation selon l'une quelconque des revendications précédentes pour l'emploi systémique à des fins d'application par voie orale, transdermique, intraveineuse, sous-cutanée, intracutanée, intramusculaire, rectale, vaginale ou sublinguale.

15. Utilisation selon l'une quelconque des revendications précédentes, pour l'emploi local sous la forme par exemple de crèmes, d'onguents, de pâtes, de gels, de solutions, de sprays, de liposomes respectivement de nanosomes, de mélanges à agiter, de pansements contenant des hydrocolloïdes respectivement d'autres substrats/véhicules dermatologiques, y compris une application par instillation.
